# EUROPÄISCHE PATENTSCHRIFT

(11) **EP 2 465 551 B1**
(45) Veröffentlichungstag und Bekanntmachung des Hinweises auf die Patenterteilung: **23.01.2019**
(21) Anmeldenummer: 11191735.7
(22) Anmeldetag: 02.12.2011
(51) Int. Cl.: A61L 31/06

(54) **Polymer Stent**
Polymeric stent
Stent en polymère

(30) Priorität: 15.12.2010 US 423124 P
(43) Veröffentlichungstag der Anmeldung: 20.06.2012
(73) Patentinhaber: Biotronik AG, 8180 Bülach (CH)
(72) Erfinder: Borck, Alexander, 91086 Aurachtal (DE)
(74) Vertreter: Randoll, Sören

(56) Entgegenhaltungen:
- WO-A1-2006/118808
- WO-A1-2010/042952
- WO-A2-2008/089434
- WO-A2-2009/142934
- US-A1- 2008 275 537
- US-A1- 2010 252 965

## Beschreibung

Die Erfindung betrifft einen Stent (eine Gefäßstütze) umfassend oder bestehend aus amorphem Polylactid. Die Erfindung betrifft ferner die Verwendung von amorphem Polylactid zur Stabilisierung eines Stents sowie ein Verfahren zum Herstellen eines entsprechenden Stents.

Polymerbasierte Stents zeigen gegenüber metallischen Stents eine geringere Festigkeit. Die minimal notwendige Radialkraft muss bei einem aus Polymeren aufgebauten Grundkörper durch mehr Material und damit größere Wandstärken ausgeglichen werden. Dickere Wandstärken und Stege (Struts) führen nachweislich zu schlechteren klinischen Resultaten z. B. aufgrund von erhöhter Neointima-Proliferation.

Aus dem Stand der Technik ist der sogenannte AMS Stent bekannt (EP 1 270 023; EP 1 632 256; EP 1 419 793). Dieser Stent besitzt Verformungseigenschaften, die zwischen denen von Polymeren und denen von Edelstahllegierungen liegen. Gleichzeitig ist der Stent im Körper abbaubar (bioresorbierbar), was für viele Anwendungen gewünscht ist.

In WO 2006/118808 A1 wird ein medizinisches Gerät gebildet aus oder beschichtet mit einem amorphen Polymer beschrieben. In WO 2009/142934 A2 wird eine semi-kristalline Zusammensetzung für die Beschichtung von implantierbaren Geräten offenbart. WO 2008/089434 A2 beschreibt biodegradierbare Endoprothesen aus amorphem Polymer. In WO 2010/042952 A1 werden bioadsorbierbare Polymere und Kompositionen daraus beschrieben. In US 2010/0252965 wird ein Verfahren zur Herstellung polymerer implantierbarer Geräte offenbart. In US 2008/0275537 A1 werden polymere Stents beschrieben.

Ferner sind biologisch abbaubare Stents auf der Basis von Polymeren bekannt. Aus Gründen der Bioverträglichkeit werden im Stand der Technik Stents aus Polylactid (PLA) eingesetzt oder Stents, die einen entsprechenden Anteil Polylactid enthalten. Polymere wie Polylactid lassen sich hinsichtlich ihres mechanischen Verhaltens in spröde, plastisch verformbar und elastisch verformbar unterteilen. Dabei ist die Umformbarkeit des Materials definiert durch die Lage der beiden folgenden Grenzen: Die Streckgrenze und die Bruchgrenze.

Dabei die ist Streckgrenze ein Werkstoffkennwert, der diejenige Spannung bezeichnet, bis zu der ein Werkstoff bei einachsiger und momentfreier Zugbeanspruchung keine sichtbare plastische Verformung zeigt. Bei Überschreiten der Streckgrenze kehrt das Material nach Entlastung nicht mehr in die ursprüngliche Form zurück, sondern es verbleibt eine Probenverlängerung. Bis zu dieser Streckgrenze tritt eine elastische, reversible Verformung auf.

An der Bruchgrenze hört die Verformung auf, das Material bricht.

Ein Stent soll grundsätzlich in der Lage sein, auf eine Dilatationsvorrichtung aufgezogen zu werden, d.h. er muss crimpbar sein, und er muss gleichzeitig dilatierbar sein, um nach Einführen an seinen Bestimmungsort im Umfang eine Ausdehnung erhalten zu können, um so für eine entsprechende Lumenvergrößerung zu sorgen. Crimpbarkeit und Dilatierbarkeit setzen eine ausreichende Plastizität voraus.

Andererseits muss ein Stent auch in der Lage sein, auf ein Gefäß eine ausreichende Stützkraft ohne elastische bzw. plastische Rückverformung auszuüben (niedriger Recoil). Diese Anforderungen stehen zueinander im Widerspruch. Die Umformungseigenschaften von Werkstoffen sind Funktionen der Zeit, der Scherkräfte und der Temperatur. Geringe elastische bzw. plastische Verformung tritt nur bei spröden Materialien auf, die sich aber dann nicht ausreichend crimpen oder dilatieren lassen. Dieser Nachteil wird im Stand der Technik zum Teil durch sehr langsame Verformungsgeschwindigkeiten (beim Dilatieren) ausgeglichen.

Dies führt dazu, dass bekannte Polymerstents, insbesondere solche aus PLA, nur mit reduzierter Geschwindigkeit dilatiert werden sollten. Üblicherweise werden 2 bar in 5 Sekunden für die Druckerhöhung bei der Dilatation als obere Grenze angegeben, was von Patienten als äußerst unangenehm empfunden wird.

Grundsätzlich ist beim Einbringen eines Implantates eine schnelle Intervention wünschenswert, weil dadurch die Belastung des kardiovaskulären Systems durch eine nur kurze Unterbrechung der Blutversorgung gering gehalten wird, wie auch eine geringe Eingriffsdauer den Organismus insgesamt weniger belastet als eine längere.

Vor diesem Hintergrund war es Aufgabe der Erfindung, einen Stent anzugeben, der über gute mechanische Eigenschaften verfügt, wobei die Wanddicken (Strutdicken) in einem medizinisch akzeptablen Bereich liegen sollten. Gleichzeitig soll der Stent bei der Implantation gut handhabbar sein, insbesondere sollte die Dilatation schnell erfolgen können und der Stent ausreichend crimpbar sein.

Der Stent als Implantat sollte als mechanische Eigenschaft insbesondere eine ausreichende Stützkraft bezogen auf Festigkeit und Dauer der Stützwirkung auf das Gefäß besitzen. Ferner war es im Sinne der Aufgabenstellung bevorzugt, dass der Stent bioresorbierbar ist und besonders bevorzugt im Sinne der Aufgabenstellung war es, dass der Abbau des Stents im Zuge der Bioresorption vornehmlich in Bereichen startet, die nicht essentiell für die Radialfestigkeit des Stents sind.

Die vorbeschriebene Hauptaufgabe der Erfindung wird gelöst durch einen Stent, umfassend oder bestehend aus Polylactid, wobei der Anteil an amorphem Polylactid ≥ 80 Gew.-% beträgt, bezogen auf die Gesamtmasse des in dem Stent enthaltenen Polylactides. Dabei befindet sich das Material in einem metastabilen Zustand, wobei das Material die Fähigkeit und das Bestreben besitzt, zu rekristallisieren.

Bevorzugt umfasst ein erfindungsgemäßer Stent einen Anteil von ≥ 90 Gew.-% an amorphen Polylactid, bezogen auf die Gesamtmasse des in dem Stent enthaltenen Polylactides.

Es hat sich überraschenderweise herausgestellt, dass Stents mit einem hohen Anteil an amorphem Polylactid die im Rahmen der Aufgabenstellung formulierte Anforderungskombination aus Crimpbarkeit und Dilatierbarkeit (ausreichende Plastizität) und Stützkraft für das Gefäß in besonderer Weise erfüllen, nämlich dadurch, dass amorphes PLA im Rahmen der Dilatation (wenigstens teilweise) in kristalline Form übergeht.

Bei P(L)LA handelt es sich in den meisten Anwendungen um ein teilkristallines Polymer. Das bedeutet, dass im Regelfall 70% des Polymers in kristallinen Domänen organisiert sind, während etwa 30% amorph vorliegen. Bei üblichen Verarbeitungsformen aus einem Lösungsmittel heraus hat das PLA-Material die Möglichkeit, über die Dauer des Abdampfens sich so strukturell auszurichten, dass ein sehr hoher Anteil in kristalliner Form vorliegt. Während amorphes PLA unter Normalbedingen metastabil ist, handelt es sich bei der kristallinen Form um den thermodynamisch stabileren Zustand. Daher werden häufig, um unkontrollierte Umwandlungsprozesse zu vermeiden, Materialien aus PLA getempert, um den Anteil an thermodynamisch stabilerem kristallinen PLA zu erhöhen.

Es ist jedoch auch technisch möglich, z. B. bei der Verarbeitung aus der Schmelze, den Anteil an amorphem PLA deutlich zu erhöhen: Dies kann beispielsweise erfolgen, indem die Polymerschmelze sehr schnell abgekühlt wird (z. B. durch Abschreckung), so dass der größte Teil (bevorzugt das gesamte Material) in amorpher Form vorliegt (unterkühlte Schmelze). Dieser Zustand kann bei Raumtemperatur für mindestens 12 Monate, weiter bevorzugt 18 Monate und besonders bevorzugt 24 Monate konserviert werden. Dabei ist es nötig, das Material nicht übermäßig zu erwärmen. Die Lagerung geschieht bevorzugt bei Raumtemperatur, wobei Temperaturanstiege auf 40°C nur für wenige Stunden (< 2h) erlaubt sind. Die Luftfeuchtigkeit soll dabei die 40-45% relative Luftfeuchtigkeit nicht überschreiten. Die Erhaltungsfähigkeit dieses metastabilen Zustandes hängt unter anderem von der Polymerkettenlänge ab. Bevorzugte Polymerkettenlänge in diesem Zusammenhang sind mittlere Molekulargewichte von 150 - 700 kDa, wobei mittlere Molmassen von 200-500 kDa bevorzugt werden. Nur Kettenlängen dieser größer sind auch bis 40°C über längere Zeiträume (bis 1 Jahr) metastabil. Kurze Ketten neigen aufgrund ihrer größeren Beweglichkeit zu Reorganisation respektive Rekristallisation.

Bei einer entsprechenden Energiezufuhr kann durch Erhöhung der Polymerkettenbeweglichkeit das amorphe PLA in den kristallinen Zustand übergehen. Dieser Reorganisationsvorgang ist exotherm. Man spricht auch von Rekristallisierung. Dabei kann die Energiezufuhr thermisch (tempern) oder auch mechanisch erfolgen.

Im Rahmen der vorliegenden Beschreibung betrifft der Begriff "Polylactid" (PLA) Lactidketten, bei denen die Monomereinheiten sämtlich die L-Form oder die D-Form des Lactides haben, oder in einer beliebigen Mischung der L- und D-Form bis einschließlich des Racemates vorliegen können. Bevorzugt im Sinne der Erfindung ist PLA als die L-Form (PLLA). Der Vorteil von PLLA ist insbesondere in einer hervorragenden Bioresorbierbarkeit sowie einer guten kommerziellen Verfügbarkeit zu sehen. Die Verwendung nur eines Stereoisomers stellt sicher, daß das Material die Fähigkeit der Rekristallisation besitzt.

Der erfindungsgemäße Stent ist in seinem Anfangszustand flexibel und leicht plastisch verformbar aufgrund des hohen Anteils an amorphen PLA. Nach der Dilatation erfährt der Stent eine strukturelle Veränderung, die vornehmlich an den während der Dilatation mechanisch belasteten Bereichen auftritt. Aufgrund der in den Stent bei der Dilatation eingebrachten mechanischen Energie geht insbesondere in diesen Bereichen ein erheblicher Teil des amorphen PLAs in den kristallinen Zustand über. Der kristalline Zustand bedeutet, dass diese PLA-Bereiche gegenüber dem amorphen Zustand verspröden, so dass Material lokal weniger flexibel wird und dadurch eine verbesserte Stützwirkung auf das Gefäß ausüben kann. Dies bedeutet, dass der erfindungsgemäße Stent vor der mechanischen Belastung bei der Dilatation plastisch leicht verformbar ist; nach der Dilatation verliert er jedoch durch Übergang von Anteilen der PLA in den kristallinen Zustand diese Fähigkeit (wenigstens teilweise), übt dafür aber eine verbesserte Stützwirkung aus.

Ein weiterer Vorteil in der erfindungsgemäßen Ausgestaltung des Stents ist darin zu sehen, dass auch eine "Nachversprödung" einsetzen kann: Bereiche des Stents, die während der Dilatation keiner starken mechanischen Belastung ausgesetzt sind, können noch erhebliche Anteile an amorphen PLA umfassen. Sofern diese Bereiche während des bestimmungsgemäßen Einsatzes einer deutlichen mechanischen Belastung ausgesetzt werden, kann durch die dadurch eingetragene Energie ein Übergang weiter Anteile des PLA in den kristallinen Zustand ausgelöst werden, was die Stützwirkung an den mechanisch besonders beanspruchten Stellen verstärkt.

Es ist bevorzugt, dass der erfindungsgemäße Stent bioresorbierbar ist. Bioresorbierbar heißt in diesem Zusammenhang, dass der Stent innerhalb eines Zeitraumes von 1-3 Jahren vom (menschlichen) Körper unter normalen Einsatzbedingungen vollständig abgebaut werden kann.

Die Bioresorbierbarkeit kann erreicht werden durch den Einsatz entsprechender Materialien, z. B. durch eine Kombination von Lactid, insbesondere L-Lactid und bioresorbierbaren Magnesiumlegierungen, wie z. B. Legierungen wie sie in EP 1 632 256 von Gerold et al. und EP 1 419 793 von Gerold et al. beschrieben werden. Bevorzugt ist jedoch, dass der Stent überwiegend - besonders bevorzugt vollständig - aus Polylactid, insbesondere Poly-L-Lactid besteht. Weitere Bestandteile eines bevorzugten Stents können noch (bioresorbierbare) Beschichtungen sein, die die Körperverträglichkeit noch weiter verbessern und/oder Wirkstoffbeschichtungen/Einschlüsse, wie z. B. Biolimus, Sirolimus oder Paclitaxel.

Bei den erfindungsgemäß bevorzugten bioresorbierbaren Stents, insbesondere solchen, die (im Wesentlichen) aus PLLA bestehen, erweist es sich als Vorteil, dass bei der Dilatation die mechanisch besonders belasteten Abschnitte des Stents zu höheren Teilen in die kristalline Form der PLLA übergehen als das bei den mechanisch weniger belasteten der Fall ist: Auch unter normalen Bedingungen im Körper, wie z. B. bei 37°C, bleiben Bereiche, die nicht mechanisch verformt wurden, über z. B. 1 - 2 Monate amorph. Diese amorphen Bereiche zeigen eine höhere Wasseraufnahme als kristalline Bereiche. Wasser unterstützt die Degradation des Stents im Rahmen der Bioresorption. Dementsprechend beginnt die Resorption bei den erfindungsgemäßen Stents an Bereichen, die für die Erhaltung der Stützkraft weniger wichtig sind. Gleichzeitig wird das Grundgerüst flexibler.
Wasser dringt leichter in amorphe Bereiche ein. Diese zeigen auch im Vergleich zu kristallinen Domänen eine geringere Dichte. Durch die größere Wasseraufnahme beziehungsweise das größere Quellungsverhalten startet der hydrolytische Abbau. Durch die Kettenbrüche, Polymerkettenverkürzung, wird das Material flexibler.

Bevorzugt ist ein erfindungsgemäßer Stent, der sterilisiert ist. Steril bedeutet in diesem Zusammenhang ein nach dem Medizingerätegesetz geeignetes Verfahren, um das Implantat im oder am menschlichen Körper zum Einsatz bringen zu dürfen.

Hierbei ist zu beachten, dass die Sterilisation erfolgen muss, ohne dass ein zu großer Anteil des amorphen Polylactides in die kristalline Form übergeht. Dementsprechend ist im Regelfall von einer Sterilisation mit Ethylenoxid (ETO) abzuraten. Bevorzugte Sterilisationsverfahren für einen erfindungsgemäßen Stent sind: E-Beam oder Gamma Strahlung, wobei das Implantat während des Sterilisationsvorgangs auf Eis (0°C) gekühlt werden sollte.

Selbstverständlich ist es angebracht, nur sterile Stents als Implantate zu verwenden. Wie angedeutet, ist die Sterilisation so durchzuführen, dass die besonderen Eigenschaften, insbesondere die Möglichkeit des Überganges von der amorphen Form in die kristalline Form für das Polymer erhalten bleiben.

Bevorzugt ist ein erfindungsgemäßer Stent, bei dem das Polylactid so ausgewählt ist, dass sich bei Lagerung bei 25°C für 12 Monate ≤ 10 Gew.% des amorphen Polylactids in kristallines Polylactid umwandeln.

Besonders bevorzugt ist im Zusammenhang mit dem erfindungsgemäßen lagerstabilen Stent, dass das Polylactid eine mittlere Molekulargröße von 150 - 700, bevorzugt 200 - 500 und besonders von 250 - 350 kDa besitzt. Diese Werte beziehen sich auf den fertigen Stent bzw. auf den Stent nach Sterilisation.

Bevorzugt ist ein erfindungsgemäßer Stent, wobei Polylactid herstellbar ist durch Extrusion von Polylactid mit einer inhärenten Viskosität (iv-Wert) von ≥ 3,3 dl/g und bevorzugt ≤ 4,3 dl/g. Die iv-Werte werden bei 25°C in Chlorform und einer Konzentration von 0,1% bestimmt. Die iv-Werte liegen nach Extrusion vor.

Polylactid dieser Ausgestaltung, insbesondere PLLA, lässt sich besonders gut handhaben und verleiht dem daraus gefertigten Stent besonders gute mechanische Eigenschaften.

Weiter bevorzugt ist ein erfindungsgemäßer Stent, wobei das Polylactid zu 1 - 10 Gew.%, bevorzugt zu 2 - 6 Gew.% bezogen auf die Gesamtmasse des Polylactides eine Molmasse von 400 Da - 3000 Da nach Extrusion besitzt.

Dieser "Größenblend" von Polylactid mit kleinen Lactidpolymeren/-oligomeren erweist sich als besonders gut bioresorbierbar. Zusätzlich wirken die kürzeren Polymerketten als polymere Weichmacher und erhöhen so zusätzlich die Flexibilität des Polymergerüstes. Die höhere Flexibilität der kürzeren Ketten hat zudem einen großen Einfluss auf die Rekristallisation. Durch den Blend der längeren Ketten mit kürzeren Ketten wird die Energiemenge, die nötig ist um die Rekristallisation zu starten, deutlich gesenkt.

Erfindungsgemäß bevorzugt ist auch ein Stent, der auf einen Ballonkatheter gecrimpt (aufgezogen) ist. Dieser erfindungsgemäß bevorzugte Stent (in Kombination mit dem Ballonkatheter) ist bereit für die Implantation.

Dabei ist bevorzugt, dass der Stent bei 5 - 15°C, besonders bevorzugt bei 10°C sehr langsam auf den Ballonkatheter aufgezogen wird. Dieser Vorgang dauert bevorzugt 0,5 - 3 Minuten, besonders bevorzugt 1 - 2 Minuten.

Das Aufziehen hat sehr vorsichtig zu erfolgen, da bei diesem Verarbeitungsschritt die Gefahr besteht, dass das Polymermaterial (wenigstens teilweise) durch die Verformung vorzeitig kristallisiert.

Selbstverständlich ist es für die erfindungsgemäßen Stents sinnvoll, sie unter geeigneten Bedingungen zu lagern. Zu hohe Luftfeuchtigkeit bei Temperaturen über 40°C befördern den Übergang von amorphen PLA (insbesondere PLLA) zu kristallinem PLA. In diesem Fall würde der Stent spröde werden und ließe sich nicht mit einer ausreichenden Geschwindigkeit dilatieren.

Bevorzugt ist dementsprechend ein erfindungsgemäßer Stent, der sich beim Einsatz als Implantat in einem Menschen oder Tier nach Einbringen in den Körper bei einer Drucksteigerung von ≥ 5 bar pro 5 Sekunden, bevorzugt ≥ 7 bar pro 5 Sekunden und besonders bevorzugt ≥ 10 bar pro 5 Sekunden dilatieren lässt.

Diese Eigenschaft kann erfindungsgemäß durch einen ausreichend hohen Anteil P(L)LA im amorphen Zustand erreicht werden, wobei bevorzugt der Stent - wie oben beschrieben - (im Wesentlichen) aus PLLA besteht.

Selbstverständlich sind die erfindungsgemäßen Stents geeignet, um im Bereich der Medizin eingesetzt zu werden. Dies gilt insbesondere für ihre bevorzugten Varianten und für den Einsatz als Implantat.

Teil der Erfindung ist auch die Verwendung von amorphem Polylactid zur Stabilisierung eines Stents durch Übergang von der amorphen Form in die kristalline Form während der Dilatation des Stents.

Durch diese Verwendung wird von den besonderen Eigenschaften der unterschiedlichen Zustände des Polylactides für den Einsatz eines Stents in vorteilhafter Weise Gebrauch gemacht. Auf diese Art können die oben beschriebenen Vorteile hinsichtlich der mechanischen Eigenschaften des Stents erreicht werden.

Die Erfindung betrifft ferner ein Verfahren zum Herstellen eines erfindungsgemäßen Stents, umfassend die Schritte:
a) Bereitstellen von Polylactid, wobei das Polylactid wenigstens zu ≥ 80 Gew.% bezogen auf die Gesamtmasse Polylactides in amorpher Form vorliegt und
b) Formen eines Stents aus dem Polylactid oder unter Verwendung des Polylactides unter solchen Bedingungen, dass der Anteil an amorphem Polylactid ≥ 80 Gew.% beträgt, bezogen auf die Gesamtmasse des in dem Stent enthaltenen Polylactides.

Mit diesem erfindungsgemäßen Verfahren lassen sich die erfindungsgemäßen Stents herstellen. Bevorzugt im Rahmen dieses Verfahren ist es selbstverständlich, dass ein weiterer Schritt, nämlich die Sterilisation durchgeführt wird. Dabei ist die Sterilisation wie oben beschrieben so durchzuführen, dass ein ausreichend hoher Anteil an amorphem Polylactid erhalten bleibt.

Weiter ist im Schritt b) darauf zu achten, dass beim Formen des Stents nicht zu viel Energie in den Stent eingebracht, sei sie mechanischer oder thermischer Natur. Dementsprechend bietet es sich, an, einen Laserschneidprozess zur Ausbildung des Stents (in seiner nicht-dilatierten Form) mittels eines UV-Lasers durchzuführen, wobei der UV-Laser bevorzugt ein Femtolaser ist.

Moderne UV-Laser sind in der Lage, Laserenergien von bis zu einem J/Puls bereitzustellen, wobei einige Laser Pulsraten von bis zu 300 Hz erreichen. Geräte mit Pulsraten von 50 Hz sind für das erfindungsgemäße Verfahren bevorzugt geeignet.

Für Zwecke der Erfindung kann der Anteil an amorphen Polymer bzw. kristallinen Polymer mittels dynamischer Differenzkalorimetrie (DSC) ermittelt werden.

Durch dem mit einer Wärmetönung verbundenen Phasenübergang von amorph zu kristallin kann das Polymer gut charakterisiert werden. Durch eine Heizrampe lässt sich der Anteil an Restkristallinität bestimmen, wenn das Material in seinen thermodynamisch stabilen Zustand übergeht. Wird das Material in der DSC geschmolzen und extrem schnell abgekühlt (>50 K/Min), lässt sich der maximale Anteil an Kristallisationswärme bestimmen, die dem Material innewohnt (also bei maximal amorphem Zustand).

Viskositätsmessungen für die oben angegebenen Viskositätswerte werden unter folgenden Bedingungen durchgeführt: Messung in Chloroform bei 25°C mit einer 0,1%ig Polymerlösung.

Nachfolgend wird die Erfindung anhand der Figuren und anhand eines Beispieles weiter erläutert:
- Fig. 1: stellt einen Wärmeflussverlauf in Abhängigkeit von der Temperatur mittels dynamischer Differenzkalorimetrie (DSC) einer amorphen Polymerprobe dar, die nach der Extrusion schnell abgekühlt wurde (Abkühlrate 50 °C/Min.)
- Fig. 2: stellt ein entsprechendes Diagramm des gleichen Materials vor, das zuvor mechanisch verformt wurde. Die Verformung des Materials betrug 300 - 400 % Längendehnung.

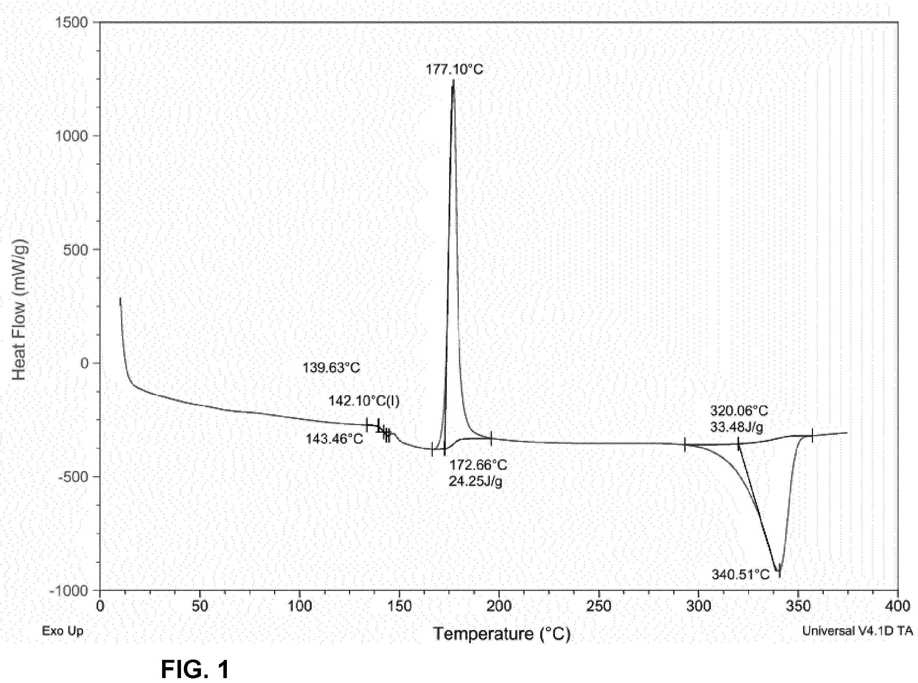

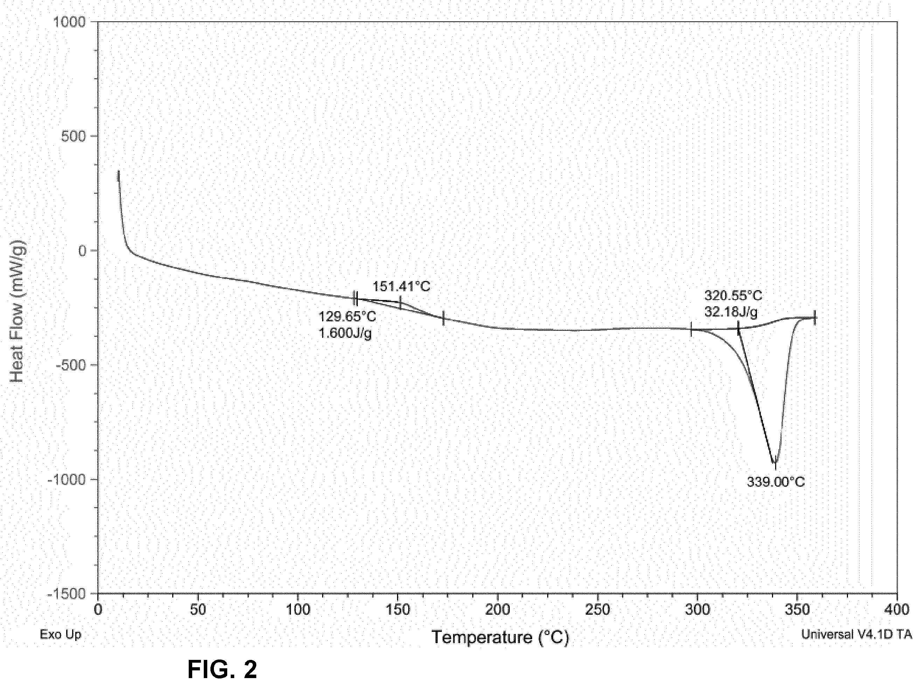

Durch den Vergleich der beiden Diagramme zueinander ist der Einfluss der mechanischen Belastung auf amorphes Material anhand von DSC-Messungen deutlich erkennbar:
Amorphe Polymere wie PLLA zeigen im Thermogramm einen exothermen Rekristallisationspeak. Wird dagegen das Material getempert bzw. so behandelt, dass ein thermostabiler Zustand erreicht wurde, so erhält man im ersten Lauf (1. Ausheizphase) keinen exothermen Peak und damit keine Rekristallisation. Dies ist in den beiden Beispielfiguren deutlich zu erkennen: In der Figur 1 ist bei 142°C der Glasübergang des Polymers zuerkennen, bei 177°C die Rekristallisation des ursprünglichen amorphen Polymers in Form eines steilen Peaks und bei 320 °C der Schmelzvorgang. Im Gegensatz zu Fig. 1 zeigt Fig. 2 einen Kurvenverlauf für ein Material, dass zuvor um 300 - 400% verformt wurde. Hier ist bei 151°C eine lediglich (Rest-)Rekristallisation und bei 320°C der Schmelzvorgang zu erkennen.

Die Rekristallisation ist bei 151°C fast komplett abgelaufen, man erkennt lediglich eine Restwärmetönung von 1,6 J/g. Dagegen ist das Schmelzverhalten in beiden Kurven sehr ähnlich (Schmelzvorgang bei 320°C).

Bereiche des Materials, zu dem in Fig. 2 das temperaturabhängige Wärmeflussverhalten abgebildet wurden, die nicht mechanischen Belastungen ausgesetzt waren, zeigten ein thermisches Verhalten entsprechend Fig. 1. Übergangsbereiche mit geringerer mechanischer Belastung zeigten auch bezüglich der Rekristallisation Werte, die hinsichtlich der Wärmetönung bei 150 - 170°C zwischen den Werten aus Figur 1 und 2 lagen.

### Beispiel

Aus PLLA (Charge L210, Boehringer Ingelheim) mit einer inhärenten Viskosität von 3,3 dl/g - 4,3 dl/g (gemessen 0,1%ig in Chloroform bei 25°C) wurde mittels eines Extrusionsverfahrens ein Rohr extrudiert Das Rohr hat eine Innendurchmesser von 0,6-1 mm vorzugsweise 0,8 mm. Die Wandstärke beträgt 70 - 250 µm vorzugsweise 100 - 200 µm weiter bevorzugt 150 µm. Extrusionsverfahren sind dem Fachmann bekannt.

Das extrudierte PLLA-Material umfasste 5 Gew.% an LLA-Oligomeren/Polymeren mit einer mittleren Molmasse von 1.500 g/mol.

Es wurde darauf geachtet, dass das Rohr während des Extrusionsverfahrens und nachfolgend nicht über Raumtemperatur erwärmt wurde. Nachfolgend wurde mittels eines Laserschneidprozesses mit einem Femtolaser ein Stent geformt. Beim Laserschneiden erfolgte im Bereich der Schnitte ein Wärmeeinfluss nur auf eine geringe Zone des jeweiligen Materials.

Nachfolgend wurde der Stent bei 10°C auf einen Ballonkatheter (Lekton Motion; BIOTRONIK AG) gecrimpt und mittels E-Beam sterilisiert. Das Instrument wurde während der Sterilisation auf Eis gelagert. Der Crimpvorgang dauerte 2 Minuten. Dabei wurde darauf geachtet, dass die mechanische Belastung des Stents gering blieb, so dass keine vorzeitige Kristallisierung des amorphen Materials erfolgte.

Nach dem Aufziehen auf den Ballonkatheter lag das PLLA-Material zu 90% in amorpher Form vor. Mit dem Beispielstent lässt sich eine Dilatation bei einer Druckerhöhung von 10 bar in 10 Sek. im menschlichen Körper durchführen, ohne dass es zu nachteiligen Ergebnissen kommt.

## Patentansprüche

1. Stent, umfassend oder bestehend aus Polylactid, wobei der Anteil an amorphem Polylactid ≥ 80 Gew.% beträgt, bezogen auf die Gesamtmasse des in dem Stent enthaltenen Polylactides, wobei das Polylactid extrudiert ist und wobei das Polylactid zu 1 - 10 Gew.% bezogen auf die Gesamtmasse des Polylactides eine Molmasse von 400 g/mol - 3.000 g/mol nach Extrusion besitzt.

2. Stent nach Anspruch 1, wobei der Anteil an amorphem Polylactid ≥ 90 Gew.% beträgt, bezogen auf die Gesamtmasse des in dem Stent enthaltenen Polylactides.

3. Stent nach Anspruch 1 oder 2, wobei das Polylactid eine mittlere Molekulargröße von 150 - 700, bevorzugt 200 - 500 und besonders 250 - 350 kDa besitzt.

4. Stent nach einem der vorangegangenen Ansprüche, wobei das Polylactid L-Polylactid ist.

5. Stent nach einem der vorangehenden Ansprüche, wobei der Stent bioresorbierbar ist.

6. Stent nach einem der vorangehenden Ansprüche, wobei der Stent steril ist.

7. Stent nach einem der vorangehenden Ansprüche, wobei das Polylactid herstellbar ist durch Extrusion von Polylactid mit einer inhärenten Viskosität von ≥ 3,3 dl/g und bevorzugt ≤ 4,3 dl/g.

8. Stent nach einem der vorangehenden Ansprüche, wobei das Polylactid zu 2 - 6 Gew.% bezogen auf die Gesamtmasse des Polylactides eine Molmasse von 400 g/mol - 3.000 g/mol nach Extrusion besitzt.

9. Stent nach einem der vorangehenden Ansprüche, wobei der Stent auf einen Ballonkatheter gecrimpt ist.

10. Stent nach einem der vorangehenden Ansprüche zum Einsatz im Bereich der Medizin.

11. Verwendung von amorphem Polylactid zur Stabilisierung eines Stents durch Übergang von der amorphen Form in die kristalline Form während der Dilatation des Stents.

## Claims

1. A stent, comprising or consisting of polylactide, wherein the proportion of amorphous polylactide is ≥ 80 % by weight, based on the total mass of the polylactide contained in the stent, wherein the polylactid is extruded and wherein the polylactid has 1 - 10% by weight based on the total mass of the polylactide a molar mass of 400 g/mol - 3,000 g/mol after the extrusion.

2. The stent according to claim 1, wherein the proportion of amorphous polylactide is ≥ 90 % by weight, based on the total mass of the polylactide contained in the stent.

3. The stent according to claim 1 or claim 2, wherein the polylactide has an average molecular weight of 150 - 700, preferably 200 - 500 and in particular 250 - 350 kDa.

4. The stent according to any one of the preceding claims, wherein the polylactide is L-polylactide.

5. The stent according to any one of the preceding claims, wherein the stent is bioresorbable.

6. The stent according to any one of the preceding claims, wherein the stent is sterile.

7. The stent according to any one of the preceding claims, wherein the polylactide can be produced by extrusion of polylactide with an inherent viscosity of ≥ 3.3 dl/g and preferred ≤ 4.3 dl/g.

8. The stent according to any one of the preceding claims, wherein 2 - 6% by weight of the polylactide based on the total mass of the polylactide has a molar mass of 400 g/mol - 3,000 g/mol after the extrusion.

9. The stent according to any one of the preceding claims, wherein the stent is crimped onto a balloon catheter.

10. The stent according to any one of the preceding claims for use in the field of medicine.

11. A use of amorphous polylactide for stabilizing a stent by transitioning from the amorphous form into the crystalline form during the dilatation of the stent.

## Revendications

1. Stent, comprenant ou étant constitué de polylactide, dans lequel la proportion en polylactide amorphe est ≥ 80 % en poids par rapport à la masse totale de polylactide contenu dans le stent, dans lequel le polylactide est extrudé et dans lequel le polylactide à raison de 1 à 10 % en poids par rapport à la masse totale de polylactide possède une masse molaire de 400 g/mole à 3000 g/mole après extrusion.

2. Stent selon la revendication 1, dans lequel la proportion en polylactide amorphe est ≥ 90 % en poids par rapport à la masse totale de polylactide contenue dans le stent.

3. Stent selon la revendication 1 ou 2, dans lequel le polylactide possède une masse moléculaire moyenne de 150 à 700, de préférence de 200 à 500, et particulièrement de 250 à 350 kDa.

4. Stent selon l'une des revendications précédentes, dans lequel le polylactide est du polylactide L.

5. Stent selon l'une des revendications précédentes, où le stent est bio-résorbable.

6. Stent selon l'une des revendications précédentes, où le stent est stérile.

7. Stent selon l'une des revendications précédentes, dans lequel le polylactide peut être fabriqué par extrusion de polylactide avec une viscosité intrinsèque >3,3 dl/g, et de préférence ≤ 4,3 dl/g.

8. Stent selon l'une des revendications précédentes, dans lequel le polylactide à 2 à 6 % en poids par rapport à la masse totale de polylactide possède une masse molaire de 400 g/mole à 3000 g/mole après extrusion.

9. Stent selon l'une des revendications précédentes, où le stent est serti sur un cathéter à ballon.

10. Stent selon l'une des revendications précédentes destiné à un emploi dans le domaine médical.

11. Utilisation de polylactide amorphe pour la stabilisation d'un stent par la transition de la forme amorphe vers la forme cristalline pendant la dilatation du stent.
